# EUROPÄISCHE PATENTSCHRIFT

(11) **EP 1 061 861 B1**
(45) Veröffentlichungstag und Bekanntmachung des Hinweises auf die Patenterteilung: **20.09.2006**
(21) Anmeldenummer: 99902495.3
(22) Anmeldetag: 16.02.1999
(51) Int. Cl.: A61B 17/90

(54) **SPIRALKLINGEN-INSERTIONS-INSTRUMENT**
SPIRAL-BLADE INSERTION INSTRUMENT
INSTRUMENT POUR INSERTION DE LAMES SPIROIDALES

(30) Priorität: 11.03.1998 DE 29804268 U
(43) Veröffentlichungstag der Anmeldung: 27.12.2000
(73) Patentinhaber: Synthes AG, Chur, 7002 Chur (CH); SYNTHES (U.S.A.), Paoli, PA 19301-1222 (US)
(72) Erfinder: ACKERET, Roman, CH-4310 Rheinfelden (CH); SENN, Peter, CH-4437 Waldenburg (CH); HUNGERBÜHLER, Ruth, CH-4438 Langenbruck (CH)
(74) Vertreter: Lusuardi, Werther
(86) Internationale Anmeldenummer: PCT/CH1999/000074
(87) Internationale Veröffentlichungsnummer: WO 1999/045858

(56) Entgegenhaltungen:
- US-A- 4 103 683

## Beschreibung

Die Erfindung bezieht sich auf eine Vorrichtung zum Einbringen einer Spiralklinge in einen Marknagel gemäss dem Oberbegriff der unabhängigen Patentansprüche 1 und 33.

Oft ist im Falle von Femurfrakturen eine rasche Mobilisation erforderlich. In solchen Fällen oder auch wenn das Kniegelenk schon offen ist, kann ein Marknagel von distal, das heisst vom Knie her in den Oberschenkel implantiert werden. Dabei werden solche Marknägel vielfach mit einer Spiralklinge, die lateral medial eingeführt wird, zur Fixation des Marknagels implantiert. Im Marknagel ist zu diesem Zweck eine Bohrung mit einem länglichen Querschnitt, zum Beispiel in der Form eines Schlitzes oder einer zu einem Schlüsselloch ähnlichen Form angebracht. Die Öffnung für die Spiralklinge im Marknagel ist geringfügig grösser als die Spiralklinge. Daher kann die Spiralklinge nur in bestimmten Drehwinkeln in den Marknagel eingeführt werden. Damit nur der Querschnitt der Spiralklinge den Knochen zerstört, muss sich die Spiralklinge beim Einbringen um die Längsachse drehen. Damit nun aber die längliche Öffnung im Marknagel beim Einbringen der Spiralklinge getroffen wird, muss beim Eintritt der Spiralklinge in den Marknagel diese in einer bestimmten Stellung stehen.

Bisher wurde bei der Spiralklingeninsertion der Knochen bis zum Nagel aufgebohrt, damit die richtige Stellung der Spiralklinge durch drehen am Ort gefunden werden konnte, oder das Instrument zeigte in diesem Fall die Richtung an. Da man in diesem Fall mit der Klinge schon auf dem Nagel ist, ist das möglich. Dadurch geht jedoch der Halt für die Spiralklinge im Knochen verloren.

Hier will die Erfindung Abhilfe schaffen. Der Erfindung liegt die Aufgabe zugrunde, die Positionierung der Spiralklinge so zu ermöglichen, dass die Spiralklinge bei der Montage beim Eintritt in den Marknagel einen Drehwinkel einnimmt, der sich mit der Orientierung der länglichen Öffnung im Marknagel deckt.

Die Erfindung löst die gestellte Aufgabe mit einer Vorrichtung zum Einbringen einer Spiralklinge in einen Marknagel, welche die Merkmale des Anspruchs 1 oder des Anspruchs 33 aufweist.

Durch die erfindungsgemässe Vorrichtung wird erreicht, dass der für die Montage richtige Drehwinkel und die richtige Position der Spiralklinge unabhängig von der Kondylenbreite und damit unabhängig vom Eintrittspunkt der Spiralklinge in den Knochen gegeben sind. Zudem soll mit Hilfe der Vorrichtung ermöglicht werden, dass der die Spiralklinge einbringende Dorn der Vorrichtung nur gerade so weit gegen den Knochen getrieben wird, dass dieser Dorn bei eingebrachter Spiralklinge bis an den Knochen reicht. Diese exakte Positionierung der Spiralklinge im Knochen kann mittels der erfindungsgemässen Vorrichtung auf folgende Arten erfolgen:
- von Auge, ohne Hilfsmittel, was allerdings eine grosse Öffnung um den Knochen herum bedingt;
- mit Röntgenstrahlen;
- mit Hilfe von Kameras, die nahe zum Knochen gebracht werden können und einem Bildschirm;
- der Abstand zwischen Vorrichtung und Knochen wird gemessen und aufgrund des Masses ein Feststellring an dem die Spiralklinge einbringenden Dorn als Anschlag fixiert;
- der die Spiralklinge einbringende Dorn der Vorrichtung wird vor der Montage der Spiralklinge an den Knochen gebracht und gleichzeitig ein Feststellring an dem die Spiralklinge einbringenden Dorn als Anschlag fixiert;
- eine kalibrierte Gewebeschutzhülse um den die Spiralklinge einbringenden Dorn ist in der Vorrichtung axial verschiebbar. Das an der kalibrierten Gewebeschutzhülse abgelesene Mass wird mit Hilfe eines Anschlags auf dem die Spiralklinge eingbringenden Dorn eingestellt. Der Dorn mit dem so eingestellten Anschlag wird bei eingebrachter Spiralklinge durch das Führungsrohr, in welchem der Dorn geführt wird, gestoppt, wodurch die Spiralklinge in axialer Richtung richtig plaziert wird;
- der die Spiralklinge einbringende Dorn der Vorrichtung erhält einen grösseren Durchmesser als die Spiralklinge und steht bei eingebrachter Spiralklinge am Knochen an; und
- die Gewebeschutzhülse um den die Spiralklinge einbringenden Dorn ist in der Vorrichtung axial verschiebbar und in ihrer Länge so bemessen, dass der Absatz am hinteren Ende des Dornes an dieser Gewebeschutzhülse ansteht, wenn die Spiralklinge in den Knochen eingebracht ist. Die Hülse ist in axialer Richtung in der Vorrichtung verschiebbar und in ihrer Länge so bemessen, dass sie mit ihrem vorderen Ende am Knochen aufstehen kann.

In einer bevorzugten Ausführungsart der erfindungsgemässen Vorrichtung enthält diese einen Zielbügel, der mittels einer Schraube am Marknagel befestigt werden kann. Im Aufsatz, der auf dem Zielbügel befestigt wird, ist eine Bohrung angebracht, die mit der länglichen Öffnung im Marknagel fluchtet. Koaxial zu dieser Bohrung wird im Aufsatz eine Führungshülse angebracht, in die ein Dorn koaxial gleitbar eingefügt ist. Die Spiralklinge ist am vorderen Ende des Dorns lösbar befestigt. Der Dorn ist an seiner Umfangsfläche mit zwei auf dem Umfang um 180° zueinander versetzten spiralförmigen Nuten versehen, wobei die Steigung der spiralförmigen Nuten der Steigung der Spiralklinge entspricht. Zwei Stifte sind quer zur Zentralachse der Führungshülse so in der Führungshülse angebracht, dass sie in die spiralförmigen Nuten eingreifen. Wird der Dorn in axialer Richtung durch eine in dieser Richtung wirkende Kraft bewegt, so wird dem Dorn durch die in den spiralförmigen Nuten gleitbaren Stifte eine Rotationsbewegung aufgezwungen. Da die Steigungen der spiralförmigen Nuten am Dorn der Steigung der Spiralklinge entsprechen, kann diese ohne zu verklemmen und ohne grosse Reibungsverluste in den Marknagel eingebracht werden. Durch die Position der Nocken ist die richtige Stellung der Spiralklinge in jeder Stellung der geführten Bewegung gegeben. Durch die Gestaltung des Zielbügels und des Führungsrohres ist der Abstand zwischen Marknagel und dem Ende der Spiralklinge beim Beginn der geführten Bewegung immer derselbe und dadurch der für die Montage günstige Drehwinkel der Spiralklinge unabhängig von der Kondylenbreite und damit unabhängig vom Eintrittspunkt der Spiralklinge in den Knochen. Auf dem Dorn ist am von der Spiralklinge entfernten Ende ein in Richtung der Zentralachse des Dorns verschiebbares Element angebracht, das auf dem Dorn fixierbar ist. Dieses Element dient beim Einbringen der Spiralklinge in den Marknagel für den Dorn als Endanschlag in axialer Richtung. Die Montage der Spiralklinge erfolgt mit Hilfe eines Führungsdrahtes, welcher vor dem Einbringen der Spiralklinge mit Hilfe einer Bohrbüchse im Knochen plaziert wird.

Eine weitere bevorzugte Ausführungsart der erfindungsgemässen Vorrichtung unterscheidet sich von der vorangehend beschriebenen Ausführungsform dadurch, dass auf dem Zielbügel ein fester Aufsatz als Führungshülse angebracht wird. In diese Führungshülse in Richtung des Marknagels koaxial teleskopierbar eingesetzt wird eine Gewebeschutzhülse, die mittels eines Griffes, der in einem Längsschlitz in der Führungshülse verschiebbar ist, gegen Rotation um die Längsachse der Gewebeschutzhülse gesichert wird. Markierungen auf der Gewebeschutzhülse ermöglichen eine Längeneinstellung der Gewebeschutzhülse, die auf diese Weise so eingestellt werden kann, dass die Gewebeschutzhülse beim Einbringen der Spiralklinge gerade auf dem Knochen aufsteht. Die Montage der Spiralklinge erfolgt auch bei dieser Ausführungsart mit Hilfe eines Führungsdrahtes, der durch eine Bohrbüchse, die koaxial in eine koaxial in die Führungshülse eingesetzte Nockenschutzhülse eingefügt wird, gesetzt wird. Die Nockenschutzhülse wird in die Führungshülse gesteckt, so dass beim Bohren die Nocken nicht verletzt werden. Die Länge der Spiralklinge wird dann am Führungsdraht abgelesen und die Bohrbüchse wieder entfernt. Durch die Nockenschutzhülse und die Gewebeschutzhülse wird nun die laterale Kortex mit einem Bohrer, der durch den Führungsdraht axial geführt wird, so tief aufgebohrt, bis der Bohrer mit dem dazu vorgesehenen Anschlag an der Nockenschutzhülse anschlägt. Anschliessend wird die Nockenschutzhülse entfernt, an der Markierung auf der Gewebeschutzhülse die Position abgelesen und dieser Position entsprechend das verschiebbare Element auf dem die Spiralklinge einbringenden Dorn als Anschlag gesetzt. Daraufhin wird der Dorn mit der aufgesetzten Spiralklinge so weit eingeschlagen, bis das auf dem Dorn als Anschlag fixierte Element auf der Führungshülse aufsteht und damit die Spiralklinge vollständig eingebracht ist.

Eine andere Ausführungsform der erfindungsgemässen Vorrichtung unterscheidet sich gegenüber der vorangehend beschriebenen Ausführungsform dadurch, dass die Führungshülse innerhalb der Gewebeschutzhülse und gegenüber dem Zielbügel axialfest angebracht ist. Die Führungshülse ist in der Bohrung der Gewebeschutzhülse konzentrisch zur Zentralachse gelagert und in axialer Richtung durch Stifte, welche in der Führungshülse und im Zielbügel verankert sind, fixiert. Die Gewebeschutzhülse ist gegenüber dem Zielbügel mittels einer im Zielbügel verlaufenden Feststellschraube fixierbar.

Wiederum eine andere Ausführungsform der erfindungsgemässen Vorrichtung besteht darin, dass der Dorn auf seiner Mantelfläche anstelle einer oder mehrerer spiralförmigen Nuten mit einem nicht selbsthemmenden ein- oder mehrgängigen Trapezgewinde oder einer ähnlichen Form versehen ist und in der Führungshülse ein entsprechendes Innengewinde angebracht ist.

Eine weitere Ausführungsform der erfindungsgemässen Vorrichtung besteht im Unterschied zu den vorangehend beschriebenen Ausführungsformen darin, dass das bei der Insertion der Spiralklinge als axialer Anschlag dienende und auf dem Dorn in einer axial wählbaren Position fixierbare Element mittels einer Sicherungsschraube, die radial in das Element einschraubbar ist und auf den Dorn drückt, blockiert wird.

Bei einer anderen Ausführungsform der erfindungsgemässen Vorrichtung wird das auf dem Dorn verschiebbare als Anschlag dienende Element durch eine Kupplung, die in auf dem Dorn angebrachte ringförmige Nuten eingreift, blockiert.

Bei einer weiteren Ausführungsform der erfindungsgemässen Vorrichtung sind am Anschlag, der auf dem Dorn verschiebbar ist, ein oder mehrere Kugeldruckstücke angebracht. Diese Kugeldruckstücke rasten in dafür vorgesehenen ringförmige Nuten auf dem Dorn ein und werden zur Blockierung des als Anschlag dienenden Elementes verwendet.

In einer anderen Ausführungsform der erfindungsgemässen Vorrichtung wird das als Anschlag dienende Element als Mutter ausgebildet und auf den Dorn, der mit einem entsprechenden Aussengewinde versehen ist, aufgeschraubt.

Eine andere Ausführungsform der erfindungsgemässen Vorrichtung besteht darin, dass das als Anschlag dienende Element mittels eines radial verschiebbaren Stiftes blockierbar ist. Dieser Stift greift in Löcher, die axial auf dem Dorn angebracht sind und somit kann der Anschlag auf dem Dorn verschoben werden.

Nochmals eine andere Ausführungsform der erfindungsgemässen Vorrichtung besteht darin, dass das als Anschlag dienende Element durch einen radial verlaufenden Schlitz als offener Ring ausgebildet ist und mit einer quer zu diesem Schlitz verlaufenden Schraube versehen ist, so dass das Element auf dem Dorn durch zusammenpressen blockiert wird.

Eine weitere Ausführungsform der erfindungsgemässen Vorrichtung unterscheidet sich von den vorangehend beschriebenen Ausführungsformen darin, dass der die Spiralklinge einbringende Dorn einen grösseren Durchmesser aufweist als die Spiralklinge selbst. Bei eingebrachter Spiralklinge steht der Dorn dann auf dem Knochen auf.

Die durch die Erfindung erreichten Vorteile sind im wesentlichen darin zu sehen, dass dank der erfindungsgemässen Vorrichtung die Positionierung der Spiralklinge so ermöglicht wird, dass die Spiralklinge beim Eintritt in den Marknagel einen Drehwinkel einnimmt, der sich mit der Orientierung der länglichen Öffnung im Marknagel deckt.

Durch die erfindungsgemässe Vorrichtung soll der für die Montage richtige Drehwinkel und die richtige Position der Spiralklinge unabhängig von der Kondylenbreite und damit unabhängig vom Eintrittspunkt der Spiralklinge in den Knochen gegeben sein. Daneben sorgen die Stifte, welche in die auf dem Dorn verlaufenden spiralförmigen Nuten eingreifen, dafür, dass der Dorn und damit die an seiner Spitze angebrachte Spiralklinge bei einer Bewegung in axialer Richtung auch eine Rotationsbewegung ausführen. Da die Steigung der spiralförmigen Nuten der Steigung der Spiralklinge entspricht, wird sich die Spiralklinge bei ihrer Montage in den Marknagel so drehen, dass die Position ihrer Querschnittsfläche während des ganzen Montagevorganges mit der länglichen Öffnung im Marknagel übereinstimmt. Die Spiralklinge lässt sich somit erheblich einfacher montieren.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Aufsicht auf den Knochen mit dem Marknagel und auf eine Ausführungsform der erfindungsgemässen Vorrichtung mit einem Schnitt durch die Führungshülse;
Fig. 2 eine Aufsicht auf den Knochen mit dem Marknagel und auf eine weitere Ausführungsform der erfindungsgemässen Vorrichtung;
Fig. 3 eine Aufsicht auf den Knochen mit dem Marknagel und auf eine weitere Ausführungsform der erfindungsgemässen Vorrichtung mit einem teilweisen Schnitt durch die Führungshülse;
Fig. 4 eine Aufsicht auf den Knochen mit dem Marknagel und auf eine weitere Ausführungsform der erfindungsgemässen Vorrichtung; und
Fig. 5 eine Aufsicht auf den Knochen und auf eine weitere Ausführungsform der erfindungsgemässen Vorrichtung mit einem Schnitt durch die Führungshülse und die Gewebeschutzhülse.

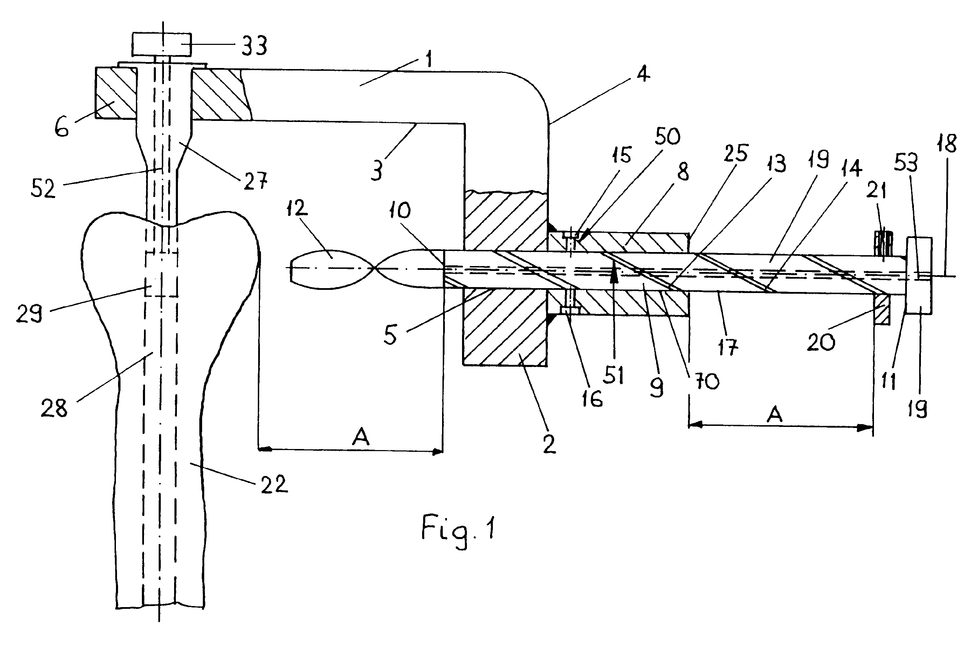

In Fig. 1 ist eine Ausführungsform der erfindungsgemässen Vorrichtung dargestellt, die einen Zielbügel 1 mit einer am einen Ende 2 des Zielbügels 1 angebrachten, von der Innenseite 3 zur Aussenseite 4 des Zielbügels 1 durchgehenden Bohrung 5, eine koaxial zur Bohrung 5 befestigte Führungshülse 8 mit der Bohrung 70 mit einer Zentralachse 18, die senkrecht zum Marknagel 28 steht, und einen in der Führungshülse 8 gleitbaren Dorn 9 umfasst. Der Zielbügel 1 ist durch eine Schraube 33 mit dem Marknagel 28 verbunden. Der in der Führungshülse 8 gleitbare Dorn 9 trifft genau auf die schlüssellochförmige Öffnung 29 im Marknagel 28, in welche eine Spiralklinge 12 eingebracht wird. Die Spiralklinge 12 ist am vorderen Ende 10 des Dorns 9 lösbar so angebracht, dass ihre möglichen Positionen für das Einbringen in den Marknagel festgelegt sind. Der Zielbügel 1 ist ein offener Winkelteil mit einer Innenfläche 3, einer Aussenfläche 4, einem vorderen Ende 6 und einem hinteren Ende 2. An seinem vorderen Ende 6 wird der Zielbügel 1 fluchtend zur Achse des Marknagels 28 über das Zielbügelrohr 27 und die Verbindungsschraube 33 mit dem Marknagel 28 verbunden. Dadurch ist der Abstand der Spiralklinge 12 vom Marknagel 28 bei Beginn der geführten Bewegung des Dornes 9 festgelegt. Die Stellung der Spiralklinge 12 ist durch Stifte 15;16 und eine spiralförmige Führung 13, 14 am Dorn 9 festgelegt. Die Abmasse der Konstruktion werden daher so gewählt, dass der Abstand der Spiralklinge 12 vom Marknagel 28 und der Drehwinkel der Spiralklinge 12, der durch die Führungshülse 8 und dem darin gleitenden Dorn 9 gegeben wird, so übereinstimmen, dass der Querschnitt der Spiralklinge 12 bei deren Berührung mit dem Marknagel 28 in dessen längliche schlüssellochförmige (runde, schlitzförmige, rechteckige) Öffnung 29 passt. Der Dorn 9 ist an seiner Umfangsfläche 17 mit zwei spiralförmigen Nuten 13;14 versehen. Zwei oder mehr Stifte 15;16 sind quer zur Zentralachse 18 in der Führungshülse 8 angebracht. Die Stifte 15;16 sind so zueinander versetzt angeordnet und ragen in die Bohrung 70 der Führungshülse 8 hinein, dass jeder Stift 15;16 in eine der spiralförmigen Nuten 13;14 eingreift. Damit wird dem Dorn 9 bei einer Bewegung in axialer Richtung eine Rotationsbewegung aufgezwungen. Die Steigungen der beiden spiralförmigen Nuten 13;14 entsprechen der Steigung der Spiralklinge 12, so dass bei einer Rotation des Dornes 9 um 360° auch die Spiralklinge 12 eine Drehung um ihre Achse um 360° ausführt. Die Stifte 15 und 16 sind in ihrer Position so angeordnet, dass die Spiralklinge 12, die auf dem Dorn 9 montiert ist, durch die Stifte 15 und 16 so geführt wird, dass die Spiralklinge 12 in die Öffnung 29 im Marknagel 28 passt. Auf dem Dorn 9 ist ein in axialer Richtung verschiebbares Element 20 angeordnet, das mittels einer radial durch das Element 20 schraubbaren Sicherungsschraube 21 in einer beliebigen axialen Position auf dem Dorn 9 fixiert werden kann. Mit Hilfe dieses fixierbaren verschiebbaren Elementes 20 kann die Bewegbarkeit des Dornes 9 in axialer Richtung so begrenzt werden, dass der von der Spiralklinge 12 zurückzulegende Weg (A) der möglichen axialen Bewegung des Dornes 9, welche durch den Abstand (A) zwischen dem fixierten Ring 20 und dem hinteren Ende 25 der Führungshülse 8 bestimmt ist, entspricht. Beim Einbringen der Spiralklinge 12 in den Knochen 22 kann der Dorn 9 nur gerade soweit gegen den Knochen 22 bewegt werden, dass die Spiralklinge 12 in den Knochen 22 und in den Marknagel 28 eingeführt wird und das vordere Ende 10 des Dornes 9 an der Aussenfläche des Knochens 22 ansteht. Die Montage der Spiralklinge 12 erfolgt mit Hilfe eines Führungsdrahtes, der vor dem Einbringen der Spiralklinge 12 mit Hilfe einer Bohrbüchse im Knochen plaziert wird und während dem Einbringen der Spiralklinge 12 durch eine im Dorn 9 speziell dafür angebrachte Bohrung 53 verläuft.

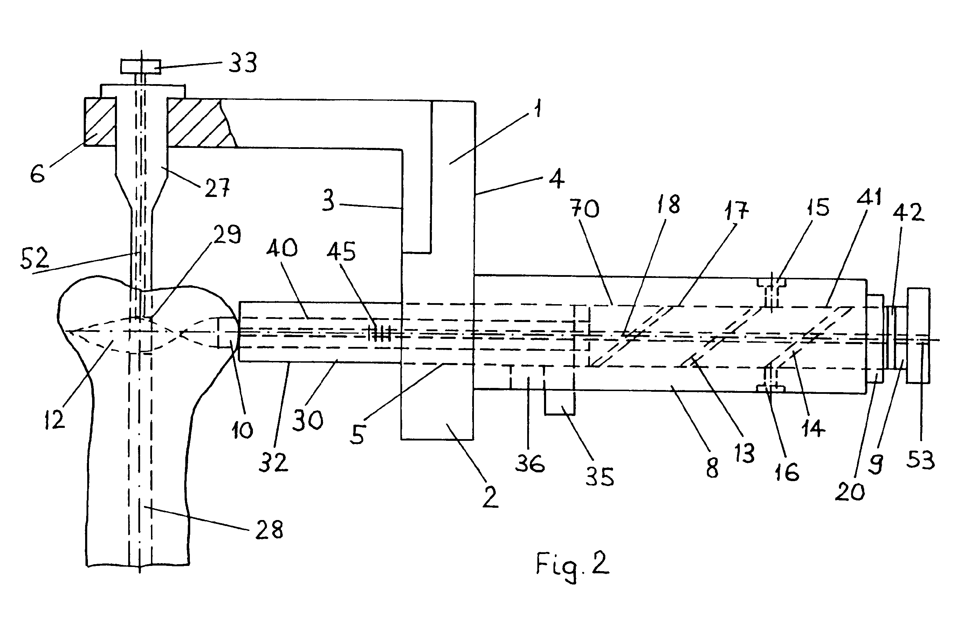

Die in Fig. 2 dargestellte Ausführungsform der erfindungsgemässen Vorrichtung unterscheidet sich von der in Fig. 1 dargestellten Ausführungsform nur dadurch, dass eine Gewebeschutzhülse 30 zum Schutz des Gewebes beim Einbringen der Spiralklinge 12 eingesetzt wird. Diese Gewebeschutzhülse 30 ist koaxial zur Zentralachse 18 gleitbar in der Führungshülse 8 gelagert und mittels eines Griffs 35, der in einem entlang der Zentralachse 18 verlaufenden Schlitz 36 in der Führungshülse 8 geführt wird, gegen Rotation um die Zentralachse 18 gesichert. Mit Hilfe dieses Griffs 35 kann die Gewebeschutzhülse 30 einfach aus dem Führungsrohr 8 entfernt werden, die Höhe kann stufenlos verstellt werden und die Gewebeschutzhülse 30 ist gesichert, dass sie nicht nach unten aus dem Zielbügel fallen kann. Auf ihrer äusseren Mantelfläche 32 ist die Gewebeschutzhülse 30 mit einer Markierung 45, die eine Längenpositionierung in Richtung der Zentralachse 18 ermöglicht, versehen. Der Dorn 9 ist in seinem Durchmesser abgestuft. Der gegen den Marknagel 28 gerichtete Teil 40 des Dornes 9 ist in seinem Durchmesser so bemessen, dass er in der Gewebeschutzhülse 30 koaxial gleitbar gelagert ist. Ein vom Marknagel 28 entfernt liegender Teil 41 des Dornes 9 hat einen grösseren, in die Bohrung 70 in der Führungshülse 8 passenden Durchmesser. Auf diesem Teil 41 des Dornes 19 sind die Spiralnuten 13;14 angebracht. Bei der in Fig. 2 gezeigten Ausführungsform der erfindungsgemässen Vorrichtung sind die Stifte 15;16 weiter entfernt vom Marknagel 28 in der Führungshülse 8 eingelassen. Auf diese Weise ist eine teleskopierbare Anordnung von Führungshülse 8 und Gewebeschutzhülse 30 möglich. Dabei kann nur die Gewebeschutzhülse 30 in der Führungshülse 8 verschoben werden. Die Führungshülse 8 hat einen fixen Abstand zum Marknagel 28. Die axiale Fixierung des verschiebbaren Elementes 20 zur Sicherung der maximalen Einbringtiefe der Spiralklinge 12 ist wie in Fig. 1 dargestellt mit einer Sicherungsschraube 21 im verschiebbaren Element 20 möglich. Im Unterschied zu der in Fig. 1 dargestellten Variante sind in der in Fig. 2 dargestellten Variante auf dem Dorn 9 noch konzentrische Rillen 42 angebracht, die eine Hilfe zur Längeneinstellung für den Weg des Dornes 9 darstellen können.

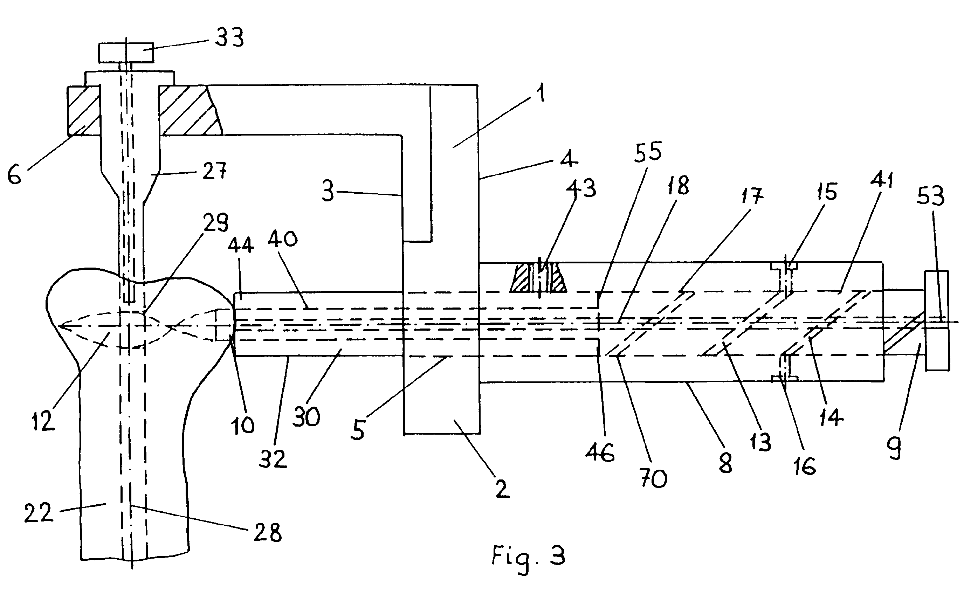

Die in Fig. 3 beschriebene Ausführungsform der erfindungsgemässen Vorrichtung unterscheidet sich von der in Fig. 2 beschriebenen Variante nur dadurch, dass die Gewebeschutzhülse 30 auch als Anschlag für den Dorn 9 beim Einbringen der Spiralklinge 12 dient. Die Gewebeschutzhülse 30 wird zu diesem Zweck vor der Montage der Spiralklinge 12 auf den Knochen angelegt und mittels einer Sicherungsschraube 43 in axialer Richtung blockiert. Der Dorn 9 weist in seinem vorderen in der Gewebeschutzhülse 30 laufenden Teil einen geringeren Durchmesser als in seinem hinteren in der Führungshülse 8 laufenden Teil 41 auf. Erreicht der hintere Teil 41 des Dornes 9 mit seinem grösseren Durchmesser die Gewebeschutzhülse 30, so steht der Dorn 9 auf der Gewebeschutzhülse 30 auf und kann in axialer Richtung nicht weiter in Richtung des Knochens gebracht werden. Der hintere, den grösseren Durchmesser aufweisende Teil des Dorns 9 ist an seiner Umfangsfläche 17 mit zwei spiralförmigen Nuten 13;14 versehen. Zwei oder mehr Stifte 15;16 sind quer zur Zentralachse 18 in der Führungshülse 8 angebracht. Die Stifte 15;16 sind so zueinander versetzt angeordnet und ragen in die Bohrung 70 der Führungshülse 8 hinein, dass jeder Stift 15;16 in eine der spiralförmigen Nuten 13;14 eingreift. Anstelle der spiralförmigen Nuten 13;14 am Dorn 9 und den Stiften 15;16 in der Führungshülse 8 kann auch ein ein- oder mehrgängiges Trapezgewinde am Dorn 9 und in der Führungshülse 8 angebracht sein. Auch damit liesse sich die schraubenförmige Bewegung erzeugen.

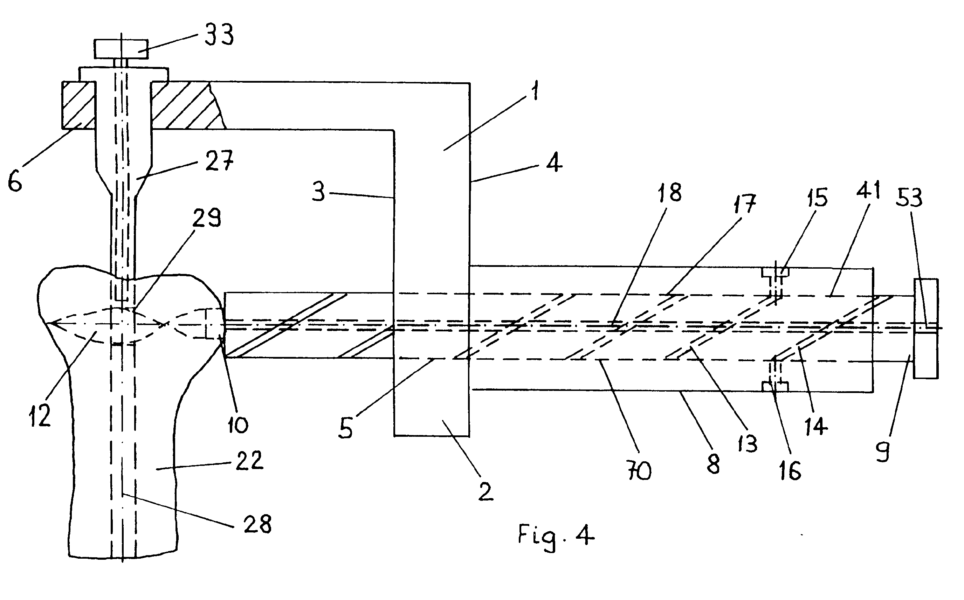

Die in Fig. 4 dargestellte Ausführungsform der erfindungsgemässen Vorrichtung unterscheidet sich in der in Fig. 1 dargestellten Ausführungsform nur dadurch, dass der Durchmesser des Dornes 9 grösser als derjenige der Spiralklinge 12 ist. Dadurch steht der Dorn 9 bei eingebrachter Spiralklinge 12 auf der Knochenoberfläche auf und erübrigt somit einen Anschlag in axialer Richtung. Analog zu der in Fig. 1 dargestellten Ausführungsform ist der Dorn 9 an seiner Umfangsfläche 17 mit zwei spiralförmigen Nuten 13;14 versehen. Zwei oder mehr Stifte 15;16 sind quer zur Zentralachse 18 in der Führungshülse 8 angebracht. Die Stifte 15;16 sind so zueinander versetzt angeordnet und ragen in die Bohrung 70 der Führungshülse 8 hinein, dass jeder Stift 15;16 in eine der spiralförmigen Nuten 13;14 eingreift. Anstelle der spiralförmigen Nuten 13;14 am Dorn 9 und den Stiften 15;16 in der Führungshülse 8 kann auch ein ein- oder mehrgängiges Trapezgewinde am Dorn 9 und in der Führungshülse 8 angebracht sein. Auch damit liesse sich die schraubenförmige Bewegung erzeugen.

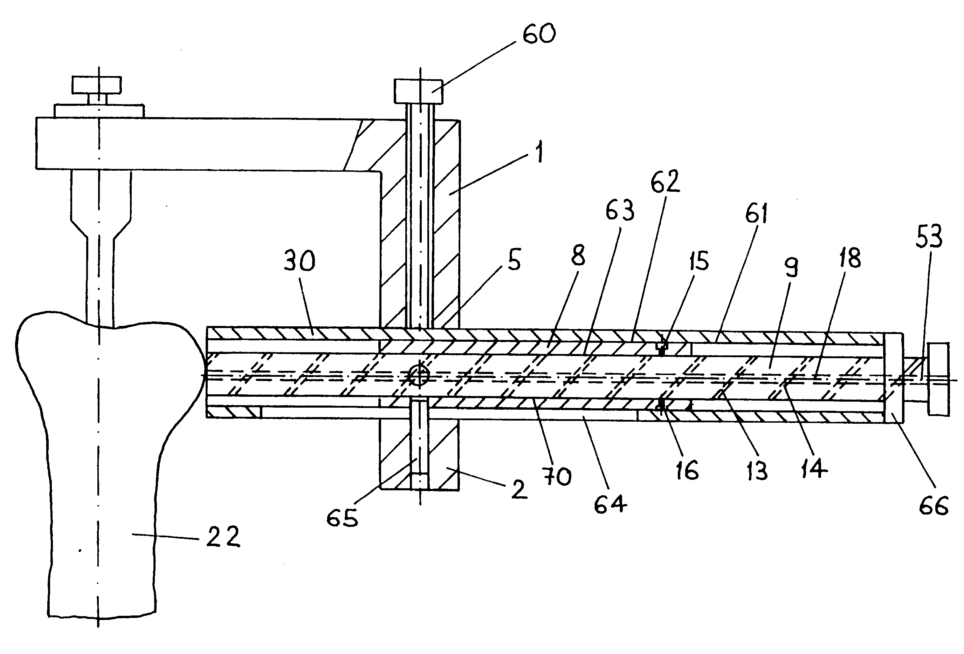

In Fig. 5 ist eine Ausführungsform der erfindungsgemässen Vorrichtung dargestellt, die sich von der in Fig. 3 gezeigten Ausführungsform nur dadurch unterscheidet, dass die Führungshülse 8 innerhalb der Gewebeschutzhülse 30 gegenüber dem Zielbügel axialfest angebracht ist. Die Gewebeschutzhülse 30 ist in der Bohrung 5 des Zielbügels 1 gleitbar gelagert und in Richtung der Zentralachse 18 in beiden Richtungen verschiebbar. Ist die Gewebeschutzhülse 30 axial in die richtige Position gebracht, kann sie mittels einer im Zielbügel 1 verlaufenden Feststellschraube 60 im Zielbügel 1 fixiert werden. Die Gewebeschutzhülse 30 ist in ihrer Länge so bemessen, dass ein auf dem Dorn 9 angebrachter fester Anschlag 66 auf dem vom Knochen 22 entfernten Ende der Gewebeschutzhülse 30 aufsteht, wenn die Spiralklinge 12 wie in Fig. 2 gezeigt in den Marknagel 28 eingebracht ist. Die Führungshülse 8 ist in der Bohrung 61 der Gewebeschutzhülse 30 konzentrisch zur Zentralachse 18 gelagert und in axialer Richtung durch Stifte 65, welche in der Führungshülse 8 und im Zielbügel 1 verankert sind, fixiert. Damit die Gewebeschutzhülse 30 in Richtung der Zentralachse 18 verschoben werden kann, ist sie mit Langlöchern 64, die als Ausnehmungen für die die Führungshülse 8 fixierenden Stifte 65 dienen, versehen. Somit kann sowohl die Gewebeschutzhülse 30 als auch der Dorn 9 in Richtung der Zentralachse 18 bewegt werden, ohne dass die Führungshülse 8 axial bewegt wird. Damit ist auch die Position der Stifte 15;16 gegenüber dem Zielbügel 1 fest. Das heisst, dass auch die Stellung der Spiralklinge 12 durch die Stifte 15;16 und die spiralförmigen Nuten 13;14 in jeder axialen Position des Dorns 9 festgelegt ist. Auch bei dieser Ausführungsform der erfindungsgemässen Vorrichtung kann anstelle der spiralförmigen Nuten 13;14 am Dorn 9 und den Stiften 15;16 in der Führungshülse 8 ein ein- oder mehrgängiges Trapezgewinde am Dorn 9 und in der Führungshülse 8 angebracht sein.

## Patentansprüche

1. Vorrichtung zum Einbringen einer Spiralklinge quer durch einen Marknagel, die folgende Elemente umfasst:
A) einen Zielbügel (1) mit einer Aussenseite (4), einer Innenseite (3) und zwei Enden (2;6), mit einer am einen Ende (2) des Zielbügels (1) angebrachten, zwischen Innenseite (3) und Aussenseite (4) durchgehenden Bohrung (5);
B) eine am Zielbügel (1) befestigte, eine Zentralachse (18) aufweisende Führungshülse (8), deren kreiszylindrische durchgehende Bohrung (70) koaxial zur Bohrung (5) verläuft; und
C) einen in der Bohrung (70) der Führungshülse (8) gleitbaren ebenfalls kreiszylindrischen Dorn (9); wobei eine Spiralklinge (12) am vorderen Ende (10) des Dorns (9) lösbar anbringbar ist,
**dadurch gekennzeichnet, dass**
der Dorn (9) auf seiner Umfangsfläche (17) und die Bohrung (70) der Führungshülse (8) mit ineinander eingreifenden Mitteln (50;51) so versehen sind, dass der Dorn (9) in der Bohrung (70) der Führungshülse (8) in Richtung der Zentralachse (18) nur schraubenförmig bewegbar ist.

2. Vorrichtung nach Anspruch 1, **dadurch gekennzeichnet, dass** die ineinandergreifenden Mittel (50;51) bezüglich des Drehwinkels um die Zentralachse (18) so positioniert sind, dass der Dorn (9), wenn er entlang der Zentralachse (18) durch die Führungshülse (8) verschoben wird und auf die die Zentralachse (18) schneidende und in einem Winkel zur Zentralachse (18) verlaufende Achse (52) des am zweiten Ende (6) des Zielbügels (1) angebrachten Zielbügelrohres (27) trifft einen für das Verriegeln geeigneten Drehwinkel einnimmt.

3. Vorrichtung nach Anspruch 1 oder 2, **dadurch gekennzeichnet, dass** sie zusätzlich eine Verbindungsschraube (33) zum Marknagel (28) umfasst, mit deren Hilfe der Zielbügel (1) am Marknagel (28) positioniert und befestigt werden kann.

4. Vorrichtung nach einem der Ansprüche 1 bis 3, **dadurch gekennzeichnet, dass** der Dorn (9) an seiner Umfangsfläche (17) mit mindestens einer spiralförmigen Nute (13;14) versehen ist und mindestens ein Stift (15;16) quer zur Zentralachse (18) so in der Führungshülse (8) angebracht ist, dass dieser in eine der spiralförmigen Nuten (13;14) eingreift.

5. Vorrichtung nach einem der Ansprüche 1 bis 3, **dadurch gekennzeichnet, dass** der Dorn (9) an seiner Umfangsfläche (17) mit einem nicht selbsthemmenden Aussengewinde versehen ist und in der Bohrung (70) der Führungshülse (8) ein entsprechendes Innengewinde angebracht ist.

6. Vorrichtung nach einem der Ansprüche 1 bis 3, **dadurch gekennzeichnet, dass** der Dorn (9) an seiner Umfangsfläche (17) mit zwei auf dem Umfang des Dorns (9) um 180° zueinander versetzten spiralförmigen Nuten (13;14) versehen ist.

7. Vorrichtung nach einem der Ansprüche 1 bis 4 oder 6, **dadurch gekennzeichnet, dass** zwei oder mehr Stifte (15;16) quer zur Zentralachse (18) auf einer beliebigen Höhe mit jeweils richtigem Winkel so zueinander versetzt in der Führungshülse (8) angebracht sind, dass jeder der Stifte (15;16) in eine der spiralförmigen Nuten (13;14) eingreift.

8. Vorrichtung nach einem der Ansprüche 1 bis 7, **dadurch gekennzeichnet, dass** die Führungshülse (8) auf der Aussenseite (4) oder der Innenseite (3) des Zielbügels (1) befestigbar ist.

9. Vorrichtung nach einem der Ansprüche 1 bis 8, **dadurch gekennzeichnet, dass** zusätzlich eine Gewebeschutzhülse (30) koaxial zur Zentralachse (18) angebracht ist.

10. Vorrichtung nach Anspruch 9, **dadurch gekennzeichnet, dass** die Gewebeschutzhülse (30) entlang der Zentralachse (18) axial verschiebbar ist.

11. Vorrichtung nach einem der Ansprüche 9 oder 10, **dadurch gekennzeichnet, dass** die Gewebeschutzhülse (30) mittels eines in einem koaxialen Schlitz (36) in der Führungshülse (8) axial bewegbaren Griffs (35) gegen Rotation um die Zentralachse (18) gesichert ist und mit Hilfe dieses Griffs (35) axial verschiebbar ist.

12. Vorrichtung nach einem der Ansprüche 9 bis 11, **dadurch gekennzeichnet, dass** die Gewebeschutzhülse (30) mit einer auf der Umfangsfläche angebrachten Markierung (45) versehen ist für eine wählbare axiale Positionierung.

13. Vorrichtung nach einem der Ansprüche 1 bis 12, **dadurch gekennzeichnet, dass** der Dorn (9) an seinem hinteren Ende (11) mit einem über den Durchmesser des Dorns (9) vorstehenden und als Anschlag dienenden Kopf (19) versehen ist.

14. Vorrichtung nach einem der Ansprüche 1 bis 13, **dadurch gekennzeichnet, dass** auf dem Dorn (9) ein in Richtung der Zentralachse (18) verschiebbares Element (20), das axial in einer beliebigen Postion fixierbar ist, angebracht ist.

15. Vorrichtung nach Anspruch 14, **dadurch gekennzeichnet, dass** das verschiebbare Element (20) durch eine radial einschraubbare Sicherungsschraube (21) auf dem Dorn (9) fixierbar ist.

16. Vorrichtung nach Anspruch 14, **dadurch gekennzeichnet, dass** das verschiebbare Element (20) radial und senkrecht zur Zentralachse (18) geschlitzt ist und durch eine senkrecht zum Schlitz verlaufende Schraube zusammengepresst wird und damit auf dem Dorn (9) fixierbar ist.

17. Vorrichtung nach Anspruch 14, **dadurch gekennzeichnet, dass** das verschiebbare Element (20) durch mindestens ein Kugeldruckstück, das in entsprechende Nuten eingreift, axial fixierbar ist.

18. Vorrichtung nach Anspruch 14, **dadurch gekennzeichnet, dass** das verschiebbare Element (20) als Mutter ausgebildet ist und über ein entsprechendes Gewinde auf dem Dorn (9) aufgeschraubt ist.

19. Vorrichtung nach Anspruch 14, **dadurch gekennzeichnet, dass** das verschiebbare Element (20) durch einen radial durch das verschiebbare Element (20) verschiebbaren und in entsprechende Löcher im Dorn (9) eingreifenden Stift axial auf dem Dorn (9) fixierbar ist.

20. Vorrichtung nach Anspruch 14, **dadurch gekennzeichnet, dass** das verschiebbare Element (20) einen radial verschiebbaren Ring enthält, welcher in eine am Dorn angebrachte Rille eingreift für eine Fixierung auf dem Dorn.

21. Vorrichtung nach einem der Ansprüche 1 bis 20, **dadurch gekennzeichnet, dass** die Führungshülse (8) zusammen mit einer Gewebeschutzhülse (30), die über die Führungshülse (8) in Richtung der Spiralklinge (12) hinausgeht, so bemessen ist, dass bei eingebrachter Spiralklinge (12) der Absatz (55) des Dornes (9) am hinteren Ende (46) der Gewebeschutzhülse (30) ansteht.

22. Vorrichtung nach einem der Ansprüche 1 bis 13, **dadurch gekennzeichnet, dass** die Führungshülse (8) in Ihrer Länge so bemessen ist, dass bei eingebrachter Spiralklinge (12) der Kopf (19) des Dornes (9) am hinteren Ende (25) der Führungshülse (8) ansteht.

23. Vorrichtung nach einem der Ansprüche 1 bis 20, **dadurch gekennzeichnet, dass** der Durchmesser des Dornes (9) grösser ist als der Durchmesser der Spiralklinge (12) für ein Anstehen des Dornes (9) bei eingedrehter Spiralklinge (12) am Knochen (22).

24. Vorrichtung nach einem der Ansprüche 1 bis 23, **dadurch gekennzeichnet, dass** zusätzlich am vorderen Ende (6) des Zielbügels (1) ein Ziel-Bohr-Gerät (27), mit dessen Hilfe eine Bohrung (27) in den Knochen (22) bohrbar ist, lösbar angebracht ist.

25. Vorrichtung nach einem der Ansprüche 1 bis 10, 13 bis 20 und 22 bis 24, **dadurch gekennzeichnet, dass** die Führungshülse (8) innerhalb der Gewebeschutzhülse (30) und gegenüber dem Zielbügel (1) axialfest angebracht ist.

26. Vorrichtung nach Anspruch 25, **dadurch gekennzeichnet, dass** die Gewebeschutzhülse (30) gegenüber dem Zielbügel (1) mittels einer im Zielbügel (1) verlaufenden Feststellschraube (60) fixierbar ist.

27. Vorrichtung nach Anspruch 25 oder 26, **dadurch gekennzeichnet, dass** die Führungshülse (8) in der Bohrung (61) der Gewebeschutzhülse (30) konzentrisch zur Zentralachse (18) gelagert und in axialer Richtung durch Stifte (65), welche in der Führungshülse (8) und im Zielbügel (1) verankert sind, fixiert ist.

28. Vorrichtung nach einem der Ansprüche 1 bis 27, **dadurch gekennzeichnet, dass** sie zusätzlich eine Spiralklinge (12) umfasst.

29. Vorrichtung nach Anspruch 28, **dadurch gekennzeichnet, dass** die Steigung der spiralförmigen Nuten (13;14) auf dem Dorn (9) der Steigung der Spiralklinge (12) entspricht.

30. Vorrichtung nach einem der Ansprüche 1 bis 29, **dadurch gekennzeichnet, dass** sie zusätzlich einen Marknagel (28) umfasst, wobei dieser Marknagel (28) durch eine Schraube (33) mit dem Zielbügel (1) verbunden ist.

31. Vorrichtung nach Anspruch 30, **dadurch gekennzeichnet, dass** die Stifte (15;16) in der Führungshülse (8) bezüglich des Drehwinkels um die Zentralachse (18) so positioniert sind, dass die Spiralklinge (12) mit dem mit den spiralförmigen Nuten (13;14) versehenen Dorn (9) durch Bewegung entlang der Zentralachse (18) in die im Marknagel (28) dafür vorgesehene Queröffnung (29) einbringbar ist.

32. Vorrichtung nach den Ansprüchen 5 und 30, **dadurch gekennzeichnet, dass** das Innengewinde in der Führungshülse (8) bezüglich des Drehwinkels um die Zentralachse (18) so positioniert ist, dass die Spiralklinge (12) mit dem mit dem Aussengewinde versehenen Dorn (9) durch Bewegung entlang der Zentralachse (18) in die im Marknagel (28) dafür vorgesehene Queröffnung (29) einbringbar ist.

33. Vorrichtung zum Einbringen einer Spiralklinge quer durch einen Marknagel, die folgende Elemente umfasst:
A) einen Zielbügel (1) mit einer Aussenseite (4), einer Innenseite (3) und zwei Enden (2;6), mit einer am einen Ende (2) des Zielbügels (1) angebrachten, zwischen Innenseite (3) und Aussenseite (4) kreiszylindrisch durchgehenden Bohrung (5), welche sich entlang einer Zentralachse (18) erstreckt; und
B) einen in der Bohrung (5) gleitbaren ebenfalls kreiszylindrischen Dorn (9); wobei eine Spiralklinge (12) am vorderen Ende (10) des Dorns (9) lösbar anbringbar ist,
**dadurch gekennzeichnet, dass**
der Dorn (9) auf seiner Umfangsfläche (17) und die Bohrung (5) mit ineinander eingreifenden Mitteln (50;51) so versehen sind, dass der Dorn (9) in der Bohrung (5) in Richtung der Zentralachse (18) nur schraubenförmig bewegbar ist.

## Claims

1. Device for inserting a spiral blade transversely through an intramedullary nail, which comprises the following elements:
A) a targeting strap (1) having an outer surface (4), an inner surface (3) and two ends (2;6), and with a through hole (5) being provided at one of the ends (2) of the targeting strap (1) passing through from the inner surface (3) to the outer surface (4);
B) a guide sleeve (8) operatively associated with the targeting strap (1) and having a central axis (18) and a circular cylindrical opening (70) running coaxially with the through hole (5); and
C) a circular cylindrical mandrel (9) slideably receivable in the opening (70) of the guide sleeve (8), whereby a spiral blade (12) is detachably mountable at the front end (10) of the mandrel (9);
**characterised in that**
mutually engaging means (50;51) are provided at the peripheral surface (17) of the mandrel (9) and in the opening (70) of the guide sleeve (8) in such manner, that the mandrel (9) is only helically moveable within the opening (70) of the guide sleeve (8) in the direction of the central axis (18).

2. Device of claim 1, wherein the mutually engaging means (50;51) are positioned with respect to the angle of rotation about the central axis (18) such, that the mandrel (9) takes up an angle of rotation being appropriate for locking, when it is moved through the guide sleeve (8) along the central axis (18) and intersects an axis (52) of the targeting strap tube (27) being attached to the second end (6) of the targeting strap (1), said axis (52) intersecting the central axis (18) and running at an angle to the central axis (18).

3. Device of claim 1 or 2, wherein it further comprises a connecting screw (33) to the intramedullary nail (28), by means of which the targeting strap (1) may be positioned and secured to the intramedullary nail (28).

4. Device of one of the claims 1 - 3, wherein the mandrel (9) is provided with at least one spiral groove (13;14) at its peripheral surface (17) and wherein at least one pin (15; 16) is attached to the guide sleeve (8) transversely to the central axis (18) such that it engages one of the spiral grooves (13;14).

5. Device of one of the claims 1 - 3, wherein the mandrel (9) is provided at its peripheral surface (17) with a non self-locking exterior thread and wherein the opening (70) of the guide sleeve (8) is provided with a mating interior thread.

6. Device of one of the claims 1 - 3, wherein the mandrel (9) comprises first and second spiral grooves (13;14) which are mutually offset by 180° on the periphery of the mandrel (9).

7. Device of one of the claims 1 - 4 or 6, wherein two or more pins (15;16) are attached to the guide sleeve (8) transversely to the central axis (18) on an arbitrary height being mutually staggered at right angles such that each of the pins (15;16) engages one of the spiral grooves (13;14).

8. Device of one of the claims 1 - 7, wherein the guide sleeve (8) is mountable at the outer surface (4) or the inner surface (3) of the targeting strap (1).

9. Device of one of the claims 1 - 8 further comprising a tissue protective sleeve (30) arranged coaxially with the central axis (18).

10. Device of claim 9, wherein the tissue protective sleeve (30) is displaceable along the central axis (18).

11. Device of claim 9 or 10, wherein the tissue protective sleeve (30) is secured against rotation about the central axis (18) by means of a handle (35) moveable within a coaxial slot (36) within the guide sleeve (8), said tissue protective sleeve (30) being axially displaceable by means of the handle (35).

12. Device of one of the claims 9 - 11, wherein the peripheral surface of the tissue protective sleeve (30) is provided with indicia (45) for selectable axial positioning.

13. Device of one of the claims 1 - 12, wherein the mandrel (9) has a head (19) at its rear end (11) protruding over the diameter of the mandrel (9) and being apt as a stop.

14. Device of one of the claims 1 - 13, wherein a displaceable element (20) being displaceable in the direction of the central axis (18) is provided on the mandrel (9), which is axially securable in an arbitrary position.

15. Device of claim 14, wherein the displaceable element (20) is fastenable on the mandrel (9) by means of a locking screw (21) being radially insertable.

16. Device of claim 14, wherein the displaceable element (20) is slit radially and perpendicularly to the central axis (18) and further being compressed by means of a screw arranged perpendicularly to the slit such being fastenable on the mandrel (9).

17. Device of claim 14, wherein the displaceable element (20) is axially arrestable by means of a ball pressing piece, which snaps corresponding grooves.

18. Device of claim 14, wherein the displaceable element (20) is configured as a nut which is screwed on a corresponding thread on the mandrel (9).

19. Device of claim 14, wherein the displaceable element (20) is axially arrestable on the mandrel (9) by means of a pin which radially passes the displaceable element (20) and fits in corresponding holes in the mandrel (9).

20. Device of claim 14, wherein the displaceable element (20) comprises a radially displaceable ring, which engages a groove placed at the mandrel for fixation on the mandrel.

21. Device of one of the claims 1 - 20, wherein the guide sleeve (8) together with a tissue protective sleeve (30),which protrudes over the guide sleeve (8) in the direction of the spiral blade (12), is dimensioned such, that when the spiral blade (12) is inserted the shoulder (55) of the mandrel (9) abuts the rear end (46) of the tissue protective sleeve (30).

22. Device of one of the claims 1 - 13, wherein the guide sleeve (8) has a length dimensioned such that when the spiral blade (12) is inserted the head (19) of the mandrel (9) abuts the rear end (25) of the guide sleeve (8).

23. Device of one of the claims 1 - 20, wherein the diameter of the mandrel (9) is greater than the diameter of the spiral blade (12) such that the mandrel (9) abuts the bone when the spiral blade (12) is inserted.

24. Device of one of the claims 1 - 23, wherein additionally a targeting strap tube (27) is loosenably mounted on the distal end (6) of the targeting strap (1), by means of which a bore may be drilled into the bone (22).

25. Device of one of the claims 1 - 10, 13 - 20 and 22 - 24, wherein the guide sleeve (8) is mounted within the tissue protective sleeve (30) and axially fixed with respect to the targeting strap (1).

26. Device of claim 25, wherein the tissue protective sleeve (30) is arrestable relative to the targeting strap (1) by means of a locking screw (60) arranged in the targeting strap (1).

27. Device of claim 25 or 26, wherein the guide sleeve (8) is accommodated concentrically to the central axis (18) in the hole (61) of the tissue protective sleeve (30) and is fixed in the axial direction by means of pins (65), which are anchored in the guide sleeve (8) and in the targeting strap (1).

28. Device of one of the claims 1 - 27 further comprising a spiral blade (12).

29. Device of claim 28, wherein the lead of the spiral shaped grooves (13;14) on the mandrel (9) correspond to the slope of the spiral blade (12).

30. Device of one of the claims 1 - 29 further comprising an intramedullary nail (28), said intramedullary nail (28) being attached to the targeting device (1) by means of a screw (33).

31. Device of claim 30, wherein the pins (15;16) in the guide sleeve (8) are positioned with respect to the angle of rotation about the central axis (18) such that the spiral blade (12) together with the mandrel (9) having the spiral grooves (13;14) is insertable into the borehole (29) being provided thereto in the intramedullary nail (28) by means of motion along the central axis (18).

32. Device of one of the claims 5 - 30, wherein the interior thread in the guide sleeve (8) - with respect to its rotational angle - is positioned such that the spiral blade (12) which is displaceable along the central axis (18) by means of the mandrel (9) being provided with the exterior thread is insertable into the borehole (29) being provided thereto in the intramedullary nail (28).

33. Device for inserting a spiral blade transversely through an intramedullary nail comprising:
A) a targeting strap (1) having an outer surface first (4), an inner surface (3) and two ends (2;6), and with a through hole (5) being provided at one of the ends (2) of the targeting strap (1) passing through from the inner surface (3) to the outer surface (4); and
B) a circular cylindrical mandrel (9) slideably receivable in the through hole (5), whereby a spiral blade (12) is detachably mountable at the front end (10) of the mandrel (9);
**characterised in that**
mutually engaging means (50;51) are provided at the peripheral surface (17) of the mandrel (9) and in the opening (70) of the guide sleeve (8) in such manner, that the mandrel (9) is only helically moveable within the through hole (5) in the direction of the central axis (18).

## Revendications

1. Dispositif pour insérer une lame hélicoïdale transversalement dans un clou intramédullaire, qui comprend les éléments suivants :
A) un viseur (1) avec une face externe (4), une face interne (3) et deux extrémités (2 ; 6), avec un trou traversant (5) entre la face interne (3) et la face externe (4), disposé au niveau de l'extrémité (2) du viseur (1) ;
B) une douille de guidage (8) présentant un axe central (18), fixée sur le viseur (1), dont le trou traversant cylindrique (70) s'étend coaxialement au trou (5) ; et
C) un mandrin également cylindrique (9) pouvant glisser dans le trou (70) de la douille de guidage (8) ; dans lequel une lame hélicoïdale (12) peut être montée de manière amovible sur l'extrémité avant (10) du mandrin (9),
**caractérisé en ce que**
le mandrin (9), sur sa circonférence (17), et le trou (70) de la douille de guidage (8) sont pourvus de moyens (50 ; 51) s'engrenant l'un dans l'autre de telle manière que le mandrin (9) n'est déplaçable que de manière hélicoïdale dans le trou (70) de la douille de guidage (8) dans le sens de l'axe central (18).

2. Dispositif selon la revendication 1, **caractérisé en ce que** les moyens s'engrenant l'un dans l'autre (50 ; 51) sont positionnés, par rapport à l'angle de rotation autour de l'axe central (18), de telle manière que le mandrin (9), lorsqu'il coulisse le long de l'axe central (18) dans la douille de guidage (8) et qu'il rencontre l'axe (52) de la tubulure du viseur (27) disposé au niveau de la deuxième extrémité (6) du viseur (1), coupant l'axe central (18) et s'étendant selon un certain angle par rapport à l'axe central (18), adopte un angle de rotation approprié pour le verrouillage.

3. Dispositif selon la revendication 1 ou 2, **caractérisé en ce qu'**il comprend en plus une vis d'assemblage (33) avec le clou intramédullaire (28), permettant de positionner et de fixer le viseur (1) sur le clou intramédullaire (28).

4. Dispositif selon l'une quelconque des revendications 1 à 3, **caractérisé en ce que** le mandrin (9) est pourvu, sur sa circonférence (17), d'au moins une rainure hélicoïdale (13 ; 14) et **en ce qu'**au moins une cheville (15 ; 16) est disposée transversalement à l'axe central (18) dans la douille de guidage (8) de telle manière que celle-ci s'engrène dans l'une des rainures hélicoïdales (13 ; 14).

5. Dispositif selon l'une quelconque des revendications 1 à 3, **caractérisé en ce que** le mandrin (9) est pourvu, sur sa circonférence (17), d'un filetage externe non autobloquant et **en ce qu'**un taraudage correspondant se trouve dans le trou (70) de la douille de guidage (8).

6. Dispositif selon l'une quelconque des revendications 1 à 3, **caractérisé en ce que** le mandrin (9) est pourvu, sur sa circonférence (17), de deux rainures hélicoïdales (13 ; 14) décalées de 180° l'une par rapport à l'autre sur la circonférence du mandrin (9).

7. Dispositif selon l'une quelconque des revendications 1 à 4 ou 6, **caractérisé en ce que** deux chevilles (15 ; 16) ou plus sont disposées dans la douille de guidage (8) transversalement à l'axe central (18), à n'importe quelle hauteur, chacune à l'angle approprié de telle manière que chacune des chevilles (15 ; 16) s'engrène dans une des rainures hélicoïdales (13; 14).

8. Dispositif selon l'une quelconque des revendications 1 à 7, **caractérisé en ce que** la douille de guidage (8) peut être fixée sur la face externe (4) ou sur la face interne (3) du viseur (1).

9. Dispositif selon l'une quelconque des revendications 1 à 8, **caractérisé en ce qu'**une douille de protection des tissus (30) est en plus disposée coaxialement à l'axe central (18).

10. Dispositif selon la revendication 9, **caractérisé en ce que** la douille de protection des tissus (30) peut coulisser axialement le long de l'axe central (18).

11. Dispositif selon l'une quelconque des revendications 9 ou 10, **caractérisé en ce que** la rotation de la douille de protection des tissus (30) autour de l'axe central (18) est bloquée au moyen d'une prise (35) axialement mobile dans une fente coaxiale (36) dans la douille de guidage (8) et **en ce que** la douille peut coulisser axialement à l'aide de cette prise (35).

12. Dispositif selon l'une quelconque des revendications 9 à 11, **caractérisé en ce que** la douille de protection des tissus (30) est pourvue d'un repère (45) placé sur la circonférence pour un positionnement axial au choix.

13. Dispositif selon l'une quelconque des revendications 1 à 12, **caractérisé en ce que** le mandrin (9) est pourvu, sur son extrémité arrière (11), d'une tête (19) dépassant du diamètre du mandrin (9) et servant de butée.

14. Dispositif selon l'une quelconque des revendications 1 à 13, **caractérisé en ce qu'**un élément (20) déplaçable dans le sens de l'axe central (18), qui peut être fixé axialement dans n'importe quelle position, est disposé sur le mandrin (9).

15. Dispositif selon la revendication 14, **caractérisé en ce que** l'élément déplaçable (20) peut être fixé sur le mandrin (9) à l'aide d'une vis d'arrêt (21) pouvant être vissée radialement.

16. Dispositif selon la revendication 14, **caractérisé en ce que** l'élément déplaçable (20) est fendu radialement et perpendiculairement à l'axe central (18) et peut être comprimé à l'aide d'une vis s'étendant perpendiculairement à la fente et être ainsi fixé sur le mandrin (9).

17. Dispositif selon la revendication 14, **caractérisé en ce que** l'élément déplaçable (20) peut être fixé axialement à l'aide d'au moins un élément de butée à billes qui s'engrène dans des rainures correspondantes.

18. Dispositif selon la revendication 14, **caractérisé en ce que** l'élément déplaçable (20) est réalisé sous forme d'écrou et peut être vissé sur le mandrin (9) par l'intermédiaire d'un filetage correspondant.

19. Dispositif selon la revendication 14, **caractérisé en ce que** l'élément déplaçable (20) peut être fixé axialement sur le mandrin (9) à l'aide d'une cheville radialement déplaçable dans l'élément déplaçable (20) et s'engrenant dans des trous correspondants dans le mandrin (9).

20. Dispositif selon la revendication 14, **caractérisé en ce que** l'élément déplaçable (20) contient une bague radialement déplaçable, laquelle s'engrène dans une gorge sur le mandrin pour une fixation sur le mandrin.

21. Dispositif selon l'une quelconque des revendications 1 à 20, **caractérisé en ce que** la douille de guidage (8) conjointement avec une douille de protection des tissus (30), qui dépasse de la douille de guidage (8) dans le sens de lame hélicoïdale (12), est dimensionnée de telle manière que lorsque la lame hélicoïdale (12) est insérée, l'épaulement (55) du mandrin (9) vient en butée avec l'extrémité arrière (46) de la douille de protection des tissus (30).

22. Dispositif selon l'une quelconque des revendications 1 à 13, **caractérisé en ce que** la longueur de la douille de guidage (8) est dimensionnée de telle manière que lorsque la lame hélicoïdale (12) est insérée, la tête (19) du mandrin (9) bute contre l'extrémité arrière (25) de la douille de guidage (8).

23. Dispositif selon l'une quelconque des revendications 1 à 20, **caractérisé en ce que** le diamètre du mandrin (9) est supérieur au diamètre de la lame hélicoïdale (12) pour que le mandrin (9) vienne en butée avec l'os (22) lorsque la lame hélicoïdale (12) est insérée.

24. Dispositif selon l'une quelconque des revendications 1 à 23, **caractérisé en ce qu'**un foret de centrage (27), permettant de percer un trou (27) dans l'os (22) est en plus disposé de manière amovible au niveau de l'extrémité avant (6) du viseur (1).

25. Dispositif selon l'une quelconque des revendications 1 à 10, 13 à 20 et 22 à 24, **caractérisé en ce que** la douille de guidage (8) est disposée à l'intérieur de la douille de protection des tissus (30) et fixement sur l'axe par rapport au viseur (1).

26. Dispositif selon la revendication 25, **caractérisé en ce que** la douille de protection des tissus (30) peut être fixée par rapport au viseur (1) à l'aide d'une vis d'ajustement fixe (60) s'étendant dans le viseur (1).

27. Dispositif selon la revendication 25 ou 26, **caractérisé en ce que** la douille de guidage (8) est logée dans l'alésage (61) de la douille de protection des tissus (30) concentriquement à l'axe central (18) et peut être fixée dans le sens axial à l'aide de chevilles (65), lesquelles sont ancrées dans la douille de guidage (8) et dans le viseur (1).

28. Dispositif selon l'une quelconque des revendications 1 à 27, **caractérisé en ce qu'**il comprend en plus une lame hélicoïdale (12).

29. Dispositif selon la revendication 28, **caractérisé en ce que** le pas des rainures hélicoïdales (13 ; 14) sur le mandrin (9) correspond au pas de la lame hélicoïdale (12).

30. Dispositif selon l'une quelconque des revendications 1 à 29, **caractérisé en ce qu'**il comprend en plus un clou intramédullaire (28), dans lequel ce clou intramédullaire (28) est relié au viseur (1) à l'aide d'une vis (33).

31. Dispositif selon la revendication 30, **caractérisé en ce que** les chevilles (15; 16) dans la douille de guidage (8) sont positionnées, par rapport à l'angle de rotation autour de l'axe central (18), de telle manière que la lame hélicoïdale (12) peut être insérée avec le mandrin (9) pourvu des rainures hélicoïdales (13 ; 14) par un mouvement le long de l'axe central (18) dans l'ouverture transversale (29) prévue à cet effet dans le clou intramédullaire (28).

32. Dispositif selon les revendications 5 et 30, **caractérisé en ce que** le taraudage dans la douille de guidage (8) est positionné, par rapport à l'angle de rotation autour de l'axe central (18), de telle manière que la lame hélicoïdale (12) peut être insérée avec le mandrin (9) pourvu du filetage externe par un mouvement le long de l'axe central (18) dans l'ouverture transversale (29) prévue à cet effet dans le clou intramédullaire (28).

33. Dispositif pour insérer une lame hélicoïdale transversalement dans un clou intramédullaire, qui comprend les éléments suivants :
A) un viseur (1) avec une face externe (4), une face interne (3) et deux extrémités (2 ; 6), avec un trou traversant (5) cylindrique entre la face interne (3) et la face externe (4), disposé au niveau de l'extrémité (2) du viseur (1), lequel trou s'étend le long d'un axe central (18) ; et
B) un mandrin (9) également circulaire pouvant glisser dans le trou (5) ; dans lequel une lame hélicoïdale (12) peut être montée de manière amovible sur l'extrémité avant (10) du mandrin (9),
**caractérisée en ce que**
le mandrin (9), sur sa circonférence (17), et le trou (5) sont pourvus de moyens (50; 51) s'engrenant l'un dans l'autre de telle manière que le mandrin (9) n'est déplaçable que de manière hélicoïdale dans le trou (5) dans le sens de l'axe central (18).
